# EUROPEAN PATENT APPLICATION

(11) **EP 3 184 031 A1**
(43) Date of publication of application: **28.06.2017**
(21) Application number: 14900173.7
(22) Date of filing: 22.08.2014
(51) Int. Cl.: A61B 5/00, A61B 5/0245, A61B 10/00, G01N 22/04

(54) **PERSPIRATION AND HEART RATE ESTIMATION SYSTEM, PERSPIRATION AND HEART RATE ESTIMATION DEVICE, AND PERSPIRATION AND HEART RATE ESTIMATION METHOD**

(71) Applicant: Fujitsu Limited, Kawasaki-shi, Kanagawa 211-8588 (JP)
(72) Inventor: YAMAJI, Takayuki, Kawasaki-shi, Kanagawa 211-8588 (JP)
(74) Representative: Hoffmann Eitle
(86) International application number: PCT/JP2014/071978
(87) International publication number: WO 2016/027360

(57) **Abstract**

A sweat and heart rate determination system includes a transmitter (1) configured to transmit a radio wave to a human body; a receiver (2) configured to receive the reflection wave of the radio wave transmitted by the transmitter (1); a heart rate determination part (34,234) configured to determine a heart rate based on a result of a frequency analysis for the reflection wave received by the receiver (2), a sweat determination part (44, 244) configured to determine a sweating state of the human body based on a strength of the reflection wave received by the receiver (2); and an output part 48, 248) configured to output information related to the heart rate determined by the heart rate determination part, and information related to the sweating state determined by the sweat determination part (44, 244).

## Description

### [Technical Field]

The present invention is related to a sweat and heart rate determination system, etc.

### [Background Art]

A moisture meter for measuring moisture of a test subject is known, which moisture meter includes a moisture measurement part of a probe type configured to be held under the armpit of the test subject to measure the moisture under the armpit for measuring the moisture content of the test subject (see Patent Document 1, for example). According to the moisture meter, the moisture measurement part transmits light from a light emitting part onto the moisture on skin under the armpit, receives reflected light thereof at a light reception part, and measures the moisture content according to a change in the received light amount.

A heart rate determination apparatus configured to emit radio waves to a test subject, detects a reflection wave from the test subject, and determines a heart rate by a frequency analysis of a detection result (see Patent Document 2, for example).

### [Citation List]

[Patent Document 1] Japanese Laid-open Patent Publication No. 2012-71056
[Patent Document 2] Japanese Laid-open Patent Publication No. 2014-039666

### [Summary]

### [Technical Problem]

However, according to the known apparatus, because of such a configuration in which the light is used to measure a moisture content and the radio waves are used to determine the heart rate, it is difficult to determine a moisture content and a heart rate with a single type of a detection wave.

Accordingly, it is an object of one aspect of the invention to provide a sweat and heart rate determination system, etc., that can determine a heart rate and a sweating state using a radio wave as a single type of a detection wave.

### [Solution to Problem]

According to an aspect of the disclosure, a sweat and heart rate determination system is provided, the sweat and heart rate determination system comprising: a transmission part configured to transmit a radio wave to a human body; a receiver configured to receive the reflection wave of the radio wave transmitted by the transmitter; a heart rate determination part configured to determine a heart rate based on a result of a frequency analysis for the reflection wave received by the receiver, a sweat determination part configured to determine a sweating state of the human body based on a strength of the reflection wave received by the receiver; and an output part configured to output information related to the heart rate determined by the heart rate determination part, and information related to the sweating state determined by the sweat determination part.

### [Advantageous Effects of Invention]

It becomes possible to obtain a sweat and heart rate determination system, etc., that can determine a heart rate and a sweating state using a radio wave as a single type of a detection wave.

### [Brief Description of Drawings]

Fig. 1 is a diagram illustrating an example of a radio wave transmission part 1 and a radio wave reception part 2.
Fig. 2 is a block diagram illustrating an example of a hardware configuration of a sweat and heart rate determination system according to an embodiment.
Fig. 3 is a block diagram illustrating an example of a radio wave transmission/reception part.
Fig. 4 is a block diagram illustrating an example of a functional configuration of a sweat and heart rate determination system 10-1.
Fig. 5 is a diagram illustrating an example of an analysis result of a frequency analysis for a detection result of data of reflection waves from a test subject 5 to which radio waves are directed.
Fig. 6 is a diagram explaining a correlation between a reflection wave strength and a sweating amount.
Fig. 7 is a flowchart illustrating an example of a sweat and heart rate determination process executed by a CPU 11 of the sweat and heart rate determination system 10-1.
Fig. 8 is a flowchart illustrating another example of a sweat and heart rate determination process executed by a CPU 11 of the sweat and heart rate determination system 10-1.
Fig. 9 is a flowchart illustrating another example of a sweat and heart rate determination process executed by a CPU 11 of the sweat and heart rate determination system 10-1.
Fig. 10 is a diagram illustrating an example of a way of holding a radio wave transmission/reception part 25.
Fig. 11 is a diagram schematically illustrating an example of a hardness 7.
Fig. 12 is a configuration diagram illustrating a sweat and heart rate determination system 10-2 according to another example.
Fig. 13 is a diagram explaining a correlation between an amplitude of a moisture sensor output and an amplitude (an amplitude of reflection waves).
Fig. 14 is a diagram illustrating a relationship between absolute humidity and an amplitude of a waveform of a Doppler sensor output.

### [Description of Embodiments]

In the following, embodiments are described in detail with reference to appended drawings.

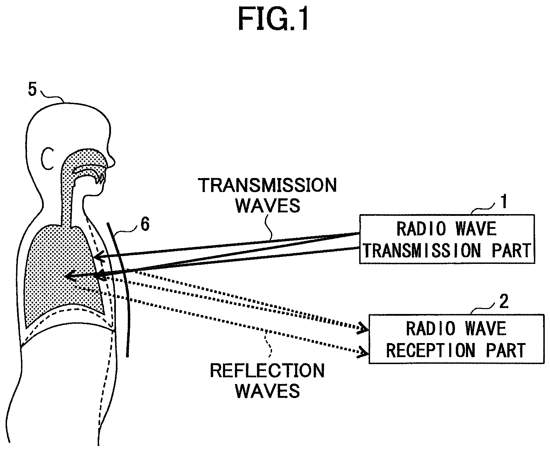

Fig. 1 is a diagram illustrating an example of a radio wave transmission part 1 and a radio wave reception part 2. A sweat and heart rate determination system 10-1 includes a radio wave transmission part 1 and a radio wave reception part 2. It is noted that, in the following, a person whose sweat and heart rate are to be determined is also referred to as "a test subject".

The radio wave transmission part 1 transmits radio waves to a human body of the test subject 5. The radio wave transmission part 1 may directly transmit the radio waves to a human body of the test subject 5; however, it is desired that the radio wave transmission part 1 transmits radio waves to a human body of the test subject 5, who wears a wear item 6, via the wear item 6. A band of a frequency of the radio wave to be used is arbitrary; however, such a frequency band is desired in which the radio waves are not harmful for the human body and difficult to be absorbed in the moisture. An example of the radio waves may be UHF(Ultra High Frequency) waves or SHF(Super High Frequency) waves. Further, the radio waves may have frequency in 2.4 GHz band, for example. The wear item 6 may be of any type of items that are formed of any material and can be on the human body. The wear item 6 may include clothes, a diaper, etc. The expression "via the wear item 6" means that there is the wear item 6 between a portion (chest, for example) of the human body on which the radio waves impinge and the radio wave transmission part 1. It is noted that, in the example illustrated in Fig. 1, the wear item 6 is an underwear item that is used such that the wear item 6 is on an upper-body of the test subject 5. The radio wave reception part 2 receives the reflection waves of the radio waves from the test subject 5 via the wear item 6. The reflection waves include a component of reflection of the radio waves at the human body of the test subject 5 and a component of reflection of the radio waves at the wear item 6.

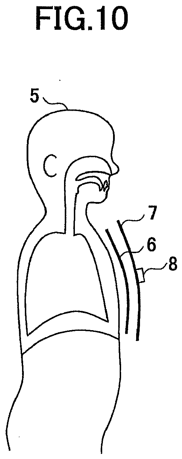

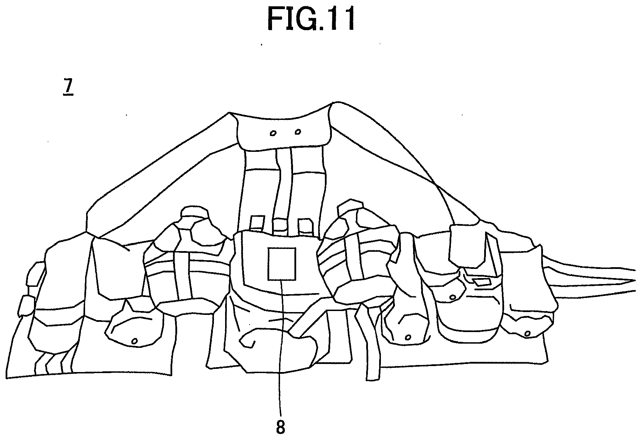

The radio wave transmission part 1 and the radio wave reception part 2 of the sweat and heart rate determination system 10-1 may be attached to an outer surface of the wear item 6 that is closest to the human body, or may be attached to another outer wear item (not illustrated) that is on the wear item 6 (see Fig. 10). For example, the radio wave transmission part 1 and the radio wave reception part 2 may be attached to a wear item called "a hardness" (see a hardness 7 in Fig. 11) that is used for training sessions of troops, etc. When the sweat and heart rate determination system 10-1 is attached to the hardness 7, the sweat and heart rate determination system 10-1 can be held at the chest of the test subject 5, in particular, a position near the heart and where the heart rate can be measured.

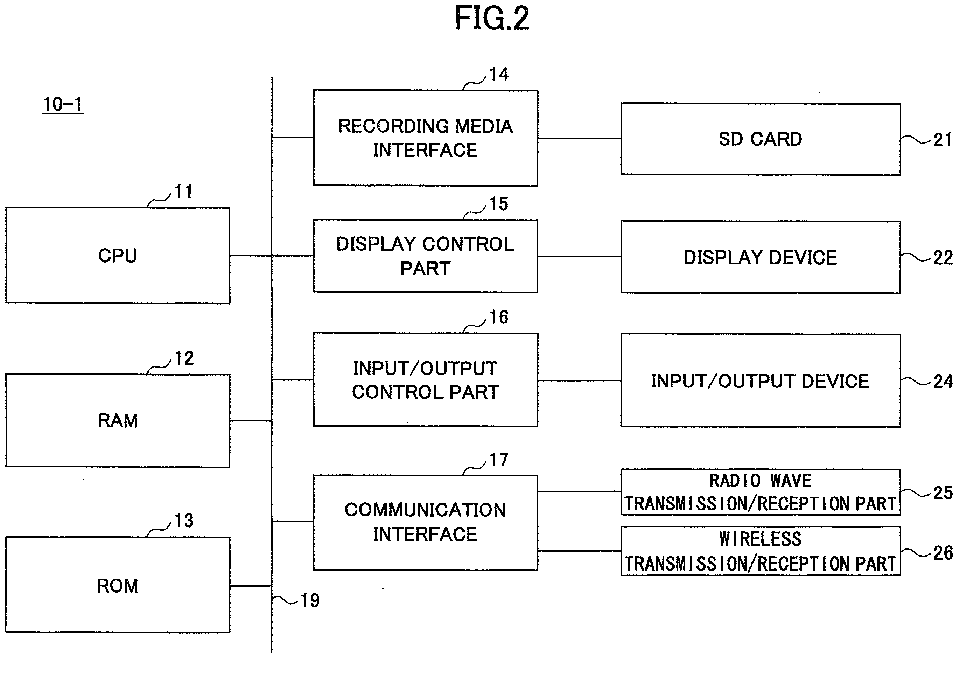

Fig. 2 is a block diagram illustrating an example of a hardware configuration of the sweat and heart rate determination system 10-1 according to an embodiment.

The sweat and heart rate determination system 10-1 includes a CPU (Central Processing Unit) 11, a RAM (Random Access Memory) 12, a ROM (Read Only Memory) 13, a recording media interface 14, and a display control part 15, coupled to each other via a bus 19, as illustrated in Fig. 2. Further, the sweat and heart rate determination system 10-1 includes an input/output control part 16 and a communication interface 17. The recording media interface 14 can be coupled to a recording medium such as a SD (Secure Digital) card (or a memory card) 21, etc. The display control part 15 is coupled to a display device 22. The input/output control part 16 is coupled to an input/output device 24. The input/output device 24 may be a touch panel, a speaker, etc. The functions of the display device 22 and the input/output device 24 may be implemented by the touch panel. It is noted that, the recording media interface 14, the SD card 21, the input/output control part 16 and the input/output device 24, the display device 22 and the display control part 15, and/or a wireless transmission/reception part 26 may be omitted, if appropriate.

The communication interface 17 is coupled to a radio wave transmission/reception part 25 and the wireless transmission/reception part 26. The radio wave transmission/reception part 25 includes the radio wave transmission part 1 and the radio wave reception part 2 illustrated in Fig. 1. The wireless transmission/reception part 26 includes a transmission/reception part that is capable of communicating via a wireless communication network used by cellular phones. The wireless transmission/reception part 26 may include a Near Field Communication (NFC) part, a Bluetooth (trademark) communication part, a Wi-Fi (Wireless-Fidelity) transmission/reception part, an infrared transmission/reception part, etc.

The CPU 11 has a function of controlling operations of the sweat and heart rate determination system 10-1 as a whole. The RAM 12 and the ROM 13 form a storage part in which programs to be executed by the CPU 11 or items of data are stored. The programs includes one or more programs that cause the CPU 11 to execute a sweat and heart rate determination process to function as a sweat and heart rate determination system. The storage part may include the SD card 21. The storage part that stores the programs is an example of a computer-readable recording medium.

The display device 22 has a function of displaying operation screens, etc., as a result of the sweat and heart rate determination process under control of the display control part 15.

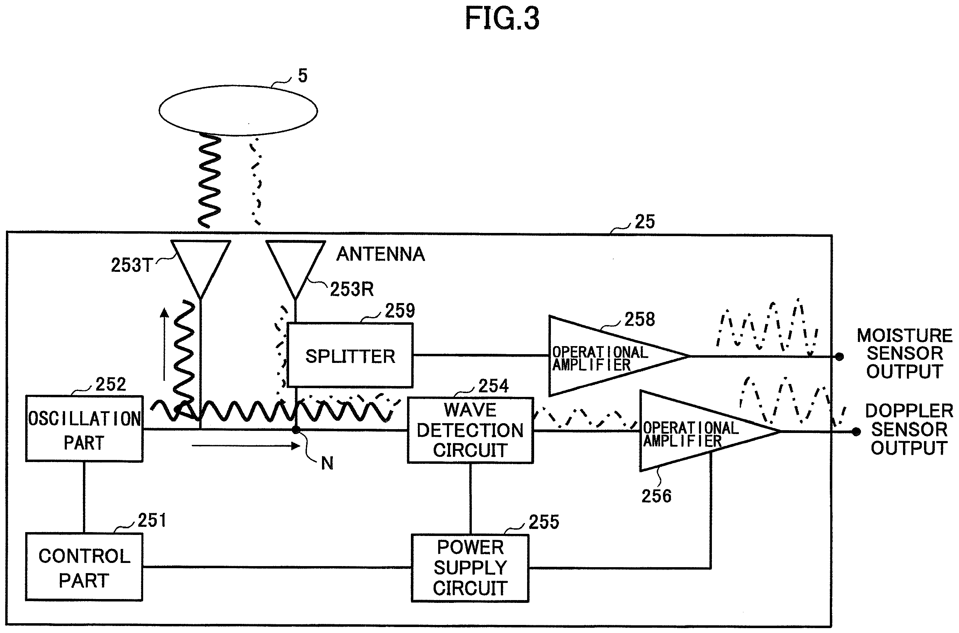

Fig. 3 is a block diagram for illustrating an example of the radio wave transmission/reception part 25.

The radio wave transmission/reception part 25 includes a control part 251, an oscillation part 252, antennas 253T, 253R, a wave detection circuit 254, a power supply circuit 255, and operational amplifiers 256, 258. Transmission waves (radio waves) generated in the oscillation part 252 are divided to be supplied to the antenna 253T and the wave detection circuit 254, and the waves transmitted from the antenna 253T impinge on the test subject 5. The waves impinging on the test subject 5 are reflected, and the reflection waves from the test subject 5 are received by the antenna 253R. The reflection waves received by the antenna 253R and indicated by alternate long and short dashed lines are divided at a splitter 259 to be supplied to a node N and the operational amplifier 258. The reflection waves supplied to the node N interferes with the transmission waves indicated by a solid line, and superposition waves (DC components) indicated by alternate long and short dashed lines are output from the wave detection circuit 254. The operational amplifier 256 inputs the amplified superposition waves to the CPU 11 via the communication interface 17 as a sensor output. The sensor output from the amplifier 256 is referred to as a "Doppler sensor output". The operational amplifier 258 inputs the amplified reflection waves to the CPU 11 via the communication interface 17 as a sensor output. The sensor output from the amplifier 258 is referred to as a "moisture sensor output".

The power supply circuit 255 includes a battery that supplies a supply voltage to the control part 251, the oscillation part 252, the wave detection circuit 254, and the operational amplifier 256. The battery is rechargeable, for example. It is noted that the power supply circuit 255 may be located outside of the radio wave transmission/reception part 25 and coupled to the radio wave transmission/reception part 25. Further, the antennas 253T, 253R may be integrated as a transmission/reception antenna.

It is noted that, in the example illustrated in Fig. 3, the radio wave transmission part 1 includes at least the oscillation part 252 and the antenna 253T, and the radio wave reception part 2 includes at least the antenna 253R, the wave detection circuit 254 and the operational amplifier 256. It is noted that, in the example illustrated in Fig. 3, the radio wave transmission/reception part 25 includes a configuration that generates the moisture sensor output; however, the configuration that generates the moisture sensor output may be omitted.

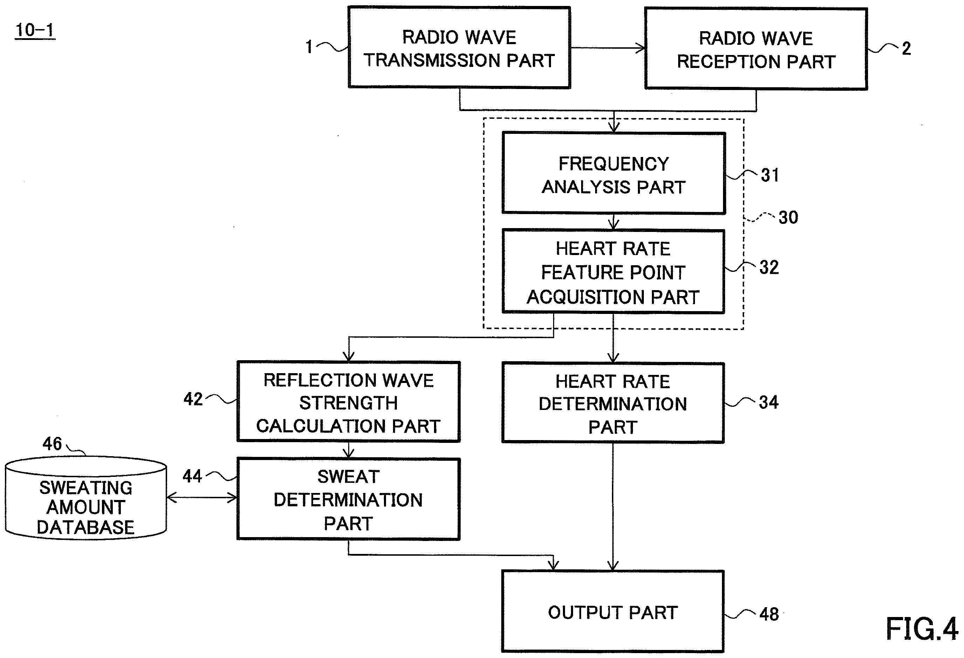

Fig. 4 is a block diagram illustrating an example of a functional configuration of the sweat and heart rate determination system 10-1.

In the example illustrated in Fig. 4, the sweat and heart rate determination system 10-1 includes the radio wave transmission part 1, the radio wave reception part 2, an analysis part 30, a heart rate determination part 34, a reflection wave strength calculation part 42, a sweat determination part 44, a sweating amount database 46, and an output part 48. The analysis part 30 includes a frequency analysis part 31 and a heart rate feature point acquisition part 32. In the example illustrated in Fig. 4, the radio wave transmission part 1 and the radio wave reception part 2 can be implemented by the radio wave transmission/reception part 25 illustrated in Fig. 2. Further, the functions of the analysis part 30, the reflection wave strength calculation part 42, the sweat determination part 44 and the output part 48 can be implemented by the CPU 11 illustrated in Fig. 2. The sweating amount database 46 can be implemented by the ROM 13 illustrated in Fig. 2. Other auxiliary storage devices such as a HDD (Hard Disk Drive), etc., may be used instead of the ROM 13.

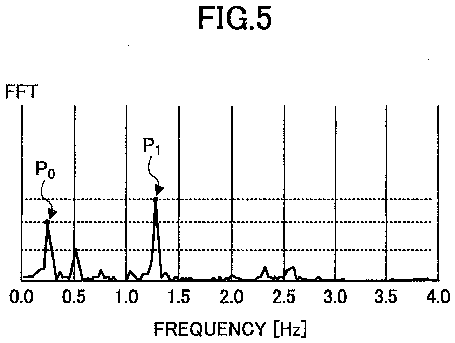

The frequency analysis part 31 performs a frequency analysis for data of the reflection waves (i.e., the Doppler sensor output) obtained from the radio wave reception part 2 to convert power spectrum of the data. The frequency analysis is FFT (Fast Fourier Transform), for example, and the analysis result illustrated in Fig. 5 is obtained. Fig. 5 is a diagram illustrating an example of an analysis result of the frequency analysis for a detection result of data of the reflection waves from the test subject 5 to which radio waves are directed. In Fig. 5, a horizontal axis represents a frequency, and a vertical axis represents a strength.

The heart rate feature point acquisition part 32 recognizes a frequency at which a peak greater than or equal to a predetermined amplitude is detected, based on the analysis result illustrated in Fig. 5. In this case, if there are a plurality of frequencies at which the peaks are detected, the heart rate feature point acquisition part 32 recognizes the frequency related to the peak corresponding to the heart rate based on a predetermined logic. Specifically, data representing the action of the test subject 5 is obtained from a microphone, an acceleration sensor, and an angular rate sensor, etc., and a peak near the frequency at which the peak is obtained by the frequency analysis of the data is removed from the peak candidates related to the heart rate. It is noted that, in the example illustrated in Fig. 5, a peak P0 corresponding to a respiration (a displacement of the body surface due to the respiration of the test subject 5) and a peak P1 corresponding to the heart rate (a displacement of the body surface due to the beat of the heart of the test subject 5, or a displacement of an organ including the heart) can be detected. The heart rate feature point acquisition part 32 may recognize the frequency related to the peak corresponding to the respiration, instead of or in addition to recognizing the frequency related to the peak corresponding to the heart rate. In this case, if there are a plurality of frequencies at which the peaks are detected, the heart rate feature point acquisition part 32 recognizes the frequency related to the peak corresponding to the respiration based on a predetermined logic. The predetermined logic is arbitrary, but may be as disclosed in Patent Document 2, for example.

Further, the heart rate feature point acquisition part 32 performs a filtering process for the data of the reflection waves such that a frequency range whose center is at the frequency of the peak recognized as the heart rate is passed. The filtering process is a Band-Pass Filter process, for example. The filtering process is performed to accurately extract the heart rate component that wanders on a beat basis. The heart rate feature point acquisition part 32 obtains a heartbeat interval (RR interval) by determining feature points obtained by a first-order differential of the filtered data. Similarly, the heart rate feature point acquisition part 32 may performs a filtering process with a center at the frequency of the peak recognized as the respiration, and obtain a respiration interval by determining feature points obtained by a first-order differential of the filtered data of the reflection waves.

The heart rate determination part 34 calculates the heart rate from the heartbeat interval. The heart rate determination part 34 may calculate a respiratory rate from the respiration interval in addition to calculating the heart rate.

The reflection wave strength calculation part 42 calculates a strength of the reflection waves (i.e., the peak value by the FFT) at the frequency of the peak recognized as the heart rate. In the following, the strength used for a determination process in the sweat determination part 44 is referred to as a "reflection wave strength". In the example, the reflection wave strength corresponds to the strength of the reflection waves at the frequency of the peak recognized as the heart rate.

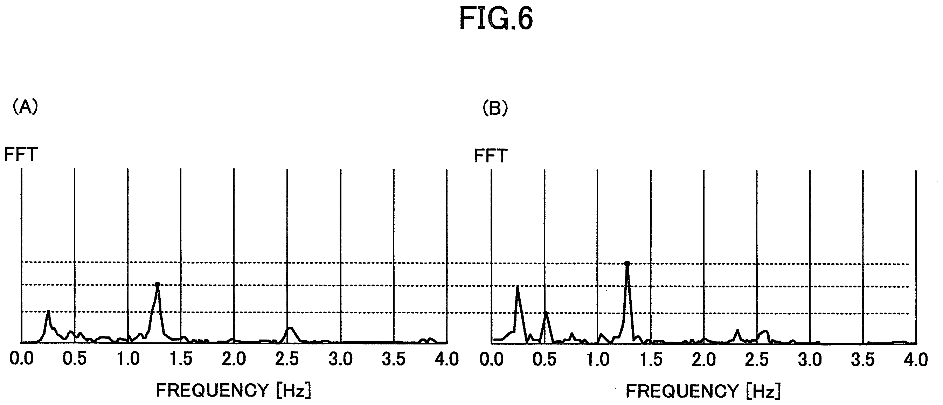

The sweat determination part 44 determines a moisture state of the human body of the test subject 5 or a moisture state of the wear item 6 based on the reflection wave strength obtained from the reflection wave strength calculation part 42. Here, the radio waves transmitted from the radio wave transmission part 1 are attenuated at the time of the reflection at the human body, and at the time of passing through the wear item 6 before the reflection waves thereof are received by the radio wave reception part 2. Specifically, when the radio waves impinge on the human body, the radio waves are absorbed by skin, muscle, fat, bone, etc. If the skin includes a large amount of the moisture content, the absorption amount becomes greater, and thus the reflection amount of the radio waves is attenuated. Thus, if the test subject 5 sweats due to the sports, etc., to have a film of the water on a surface of the skin due to the sweat (the same holds true for the water of the soaked wear item 6), the strength of the reflection signal is attenuated. For example, the radio waves in the 2.4 GHz band have the attenuation 0.4 dB at the water film of 0.1mm thickness, and the radio waves in the 24 GHz band have the attenuation 6 dB at the water film of 0.1mm thickness. In this way, the inventor of the present invention confirms that there is a correlation between the attenuation amount of the radio waves and the moisture state of the human body or the moisture state of the wear item 6 (see Fig. 6). The attenuation amount of the radio waves corresponds to the reduced amount of the reflection wave strength. Fig. 6 is a diagram explaining a relationship between absolute humidity and the reflection wave strength. In Fig 6, (A) illustrates the case of the absolute humidity being 80%, and (B) illustrates the case of the absolute humidity being 30%. In Fig. 6, a horizontal axis represents a frequency, and a vertical axis represents a strength. The absolute humidity is at the space between the human body and the wear item 6, and is an index representing the moisture state of the human body and the moisture state of the wear item 6. As illustrated in Fig. 6, the reflection wave strength becomes smaller as the absolute humidity become higher. In other words, the attenuation amount becomes greater as the absolute humidity becomes higher. The reflection wave strength and the absolute humidity can be associated with each other by a certain relationship F. The relationship F can be derived in advance with a curve fitting using a plurality of items of actual data. The relationship F may be stored in the sweating amount database 46.

The sweat determination part 44 determines a sweating amount of the human body (an example of a sweating state) based on the determined moisture state of the human body and the determined moisture state of the wear item 6. There is a correlation between the moisture state of the human body or the moisture state of the wear item 6 and the sweating amount. Basically, the moisture state of the human body or the moisture state of the wear item 6 increases as the sweating amount increases. The moisture state of the human body or the moisture state of the wear item 6 and the sweating amount can be associated with each other by a certain relationship F1. The relationship F1 may be a proportional relationship (a linear function), for example. The relationship F1 may be stored in the sweating amount database 46.

However, the sweat determination part 44 may determine the sweating amount of the human body directly based on the reflection wave strength obtained by the reflection wave strength calculation part 42. Specifically, the sweat determination part 44 may determine the sweating amount of the human body directly based on the reflection wave strength obtained by the reflection wave strength calculation part 42 without determining the moisture state of the human body or the moisture state of the wear item 6. This is because there is a correlation between the reflection wave strength and the sweating amount of the human body, which is because of a fact that there is a correlation between the reflection wave strength and the moisture state of the human body or the moisture state of the wear item 6 and there is a correlation between the moisture state of the human body or the moisture state of the wear item 6 and the sweating amount of the human body.

The sweating amount database 46 stores the relationship F that associates the reflection wave strength with the absolute humidity. Alternatively, the sweating amount database 46 may store table data of many associated pairs of the reflection wave strength and the absolute humidity. Further, the sweating amount database 46 stores the relationship F1 that associates the absolute humidity with the sweating amount. Alternatively, the sweating amount database 46 may store table data of many associated pairs of the absolute humidity and the sweating amount.

Alternatively, the sweating amount database 46 may store the relationship F2 that associates the reflection wave strength with the sweating amount. Alternatively, the sweating amount database 46 may store table data of many associated pairs of the reflection wave strength and the sweating amount.

The output part 48 outputs the heart rate determined by the heart rate determination part 34. The output part 48 may output the respiratory rate determined by the heart rate determination part 34, in addition to the heart rate. A way of outputting the heart rate determined by the heart rate determination part 34 is arbitrary. For example, the heart rate determined by the heart rate determination part 34 may be outputted on the display device 22, or may be outputted with sound messages, etc. Further, for example, when the heart rate determined by the heart rate determination part 34 exceeds a predetermined threshold, the output part 48 may transmit information representing that the determined heart rate exceeds the predetermined threshold. A way of outputting the respiratory rate may be the same.

The output part 48 outputs the sweating amount determined by the sweat determination part 44. A way of outputting the sweating amount determined by the sweat determination part 44 is arbitrary. For example, the sweating amount determined by the sweat determination part 44 may be outputted on the display device 22, or may be outputted with sound messages, etc. Further, for example, when the sweating amount determined by the sweat determination part 44 exceeds a predetermined threshold, the output part 48 may transmit information representing that the determined sweating amount exceeds the predetermined threshold.

According to the sweat and heart rate determination system 10-1 illustrated in Fig. 4, it becomes possible to determine and output not only the heart rate of the test subject 5 but also the sweating amount of the human body of the test subject 5 based on the Doppler sensor output. The Doppler sensor output is generated by the radio wave transmission part 1 and the radio wave reception part 2, as described above. Thus, according to the sweat and heart rate determination system 10-1 illustrated in Fig. 4, it becomes possible to determine and output not only the heart rate of the test subject 5 but also the sweating amount of the human body of the test subject 5 using the radio waves (i.e., a single type of a detection wave).

Further, according to the sweat and heart rate determination system 10-1 illustrated in Fig. 4, it becomes possible to determine and output not only the heart rate of the test subject 5 but also the sweating amount of the human body of the test subject 5 in a non-contact manner, because the radio waves are utilized. Unlike the light, the radio waves are not influenced by the color of the reflection surface (i.e., the skin) of the human body. Thus, according to the sweat and heart rate determination system 10-1 illustrated in Fig. 4, it becomes possible to determine the sweating amount, etc., even if the radio wave impinging location cannot be fixed in the human body due to the motion of the test subject 5, etc. Further, in the case of the measurement with the light, the light absorption factor varies according to the material of the wear item 6, and thus the amount of the reflected light varies according to the material of the wear item 6. In contrast, with respect to the light, the radio waves have the decreased change in the strength of the reflection waves due to the wear item 6. Thus, according to the sweat and heart rate determination system 10-1 illustrated in Fig. 4, it becomes possible to determine the sweating amount of the human body, regardless of the material of the wear item 6 on the human body. Further, in the case of the measurement with the light, when the sweat, the oil or the like adheres to the light emitting part or the light reception part, the amount of the reflection light is reduced. In contrast, in the case of the radio waves, even if the sweat, the oil or the like adheres to the radio wave transmission part 1 or the radio wave reception part 2, the reduced amount of the strength of the reflection waves is smaller with respect to the light. Thus, according to the sweat and heart rate determination system 10-1 illustrated in Fig. 4, it becomes possible to determine the sweating amount of the human body, even in a circumstance where the sweat, the oil or the like easily adheres thereto.

Further, according to the sweat and heart rate determination system 10-1 illustrated in Fig. 4, the sweating amount is determined utilizing the fact that there is a correlation between the attenuation amount of the radio waves and the sweating amount. Thus, according to the sweat and heart rate determination system 10-1 illustrated in Fig. 4, it becomes possible to determine the sweating amount of the human body with high accuracy.

Further, according to the sweat and heart rate determination system 10-1 illustrated in Fig. 4, the reflection wave strength calculation part 42 calculates the peak value (i.e., the reflection wave strength) related to the frequency of the heart rate feature point. The peak value related to the frequency of the heart rate feature point is derived based on the result of the FFT executed in the heart rate determination process. Thus, according to the sweat and heart rate determination system 10-1 illustrated in Fig. 4, it becomes possible to determine the sweating amount of the human body effectively utilizing the result obtained by the heart rate determination process.

Further, according to the sweat and heart rate determination system 10-1 illustrated in Fig. 4, the reflection wave strength calculation part 42 calculates the peak value related to the frequency of the heart rate feature point. The peak value related to the frequency of the heart rate feature point is great itself (see Fig. 5), which enables increasing the resolution for evaluating the reduced amount of the reflection wave strength. Thus, according to the sweat and heart rate determination system 10-1 illustrated in Fig. 4, it becomes possible to determine the sweating amount of the human body with high accuracy based on the peak value related to the frequency of the heart rate feature point.

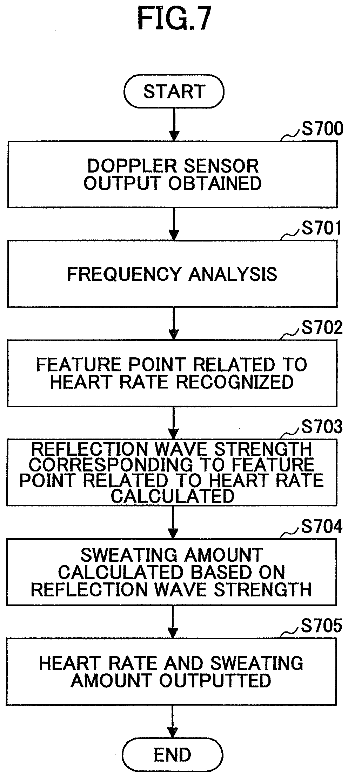

Fig. 7 is a flowchart illustrating an example of the sweat and heart rate determination process executed by the CPU 11 of the sweat and heart rate determination system 10-1. A process routine illustrated in Fig. 7 may be executed repeatedly at a predetermined cycle.

In step S700, the frequency analysis part 31 obtains the Doppler sensor output over a time period ΔT corresponding to the predetermined cycle to perform the frequency analysis.

In step S701, the heart rate feature point acquisition part 32 recognizes one or more feature points related to the heart rate based on the frequency analysis result in step S700.

In step S702, the heart rate determination part 34 determines the heart rate per a unit of time based on a plurality of the feature points related to the heart rate.

In step S703, the heart rate feature point acquisition part 42 calculates the peak value (i.e., the reflection wave strength) related to the frequency of the feature point based on the frequency analysis result in step S700. If there are a plurality of the peak values related to the heart rate over the time period ΔT, the reflection wave strength calculation part 42 may calculate the average of the peak values.

In step S704, the sweat determination part 44 calculates the sweating amount based on the reflection wave strength calculated in step S703. In this example, the sweating amount database 46 may store the relationship F2 that associates the reflection wave strength with the sweating amount. The sweat determination part 44 obtains the sweating amount by substituting the reflection wave strength calculated in step S703 into the relationship F2.

In step S705, the output part 48 simultaneously outputs the calculation result (i.e., the determination result) of the heart rate obtained in step S702 and the calculation result (i.e., the determination result) of the sweating amount obtained in step S704. A destination to which the calculation results are to be outputted is arbitrary. The destination may be a monitor (not illustrated) that monitors the test subject 5, etc., for example.

According to the sweat and heart rate determination process illustrated in Fig. 7, the heart rate determination part 34 can determine the heart rate of the test subject 5 based on the Doppler sensor output over the time period ΔT. The sweat determination part 44 can determine the sweating amount of the test subject 5 based on the Doppler sensor output over the time period ΔT. Further, the output part 48 can output the determination result of the heart rate of the test subject 5 as well as the determination result of the sweating amount of the test subject 5. The Doppler sensor output is generated by the radio wave transmission part 1 and the radio wave reception part 2, as described above. Thus, according to the sweat and heart rate determination process illustrated in Fig. 7, it becomes possible to determine and output the heart rate of the test subject 5 and the sweating amount of the test subject 5 utilizing the radio waves. Further, by utilizing the radio waves, the same effects as described above with reference to Fig. 4 can be obtained.

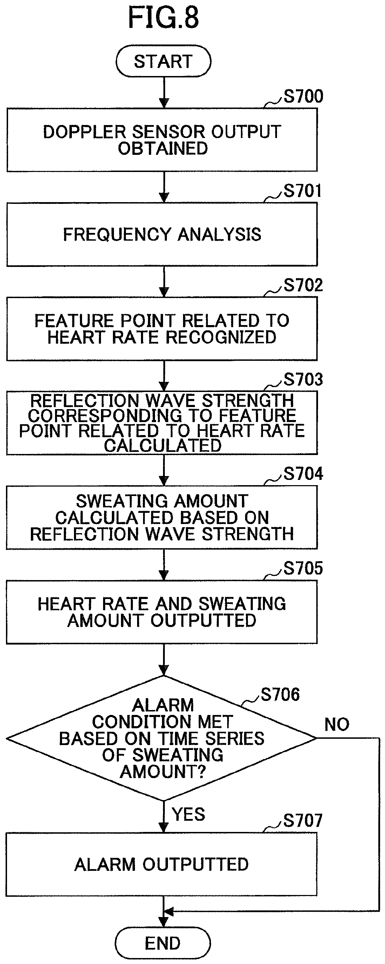

Fig. 8 is a flowchart illustrating another example of a sweat and heart rate determination process executed by the CPU 11 of the sweat and heart rate determination system 10-1. A process routine illustrated in Fig. 8 may be executed repeatedly at a predetermined cycle.

The process illustrated in Fig. 8 differs from the process illustrated in Fig. 7 in that processes after step 706 are added. In the following, the difference is described.

In step S706, the output part 48 determines, based on a history (a change in a time series) of the calculation results of the sweating amount obtained in step S704, whether an alarm condition is met. The alarm condition is arbitrary. For example, the alarm condition may be met when the determination result of the sweating amount indicates a state in which the sweating amount is not changed after indicating a state in which the sweating amount has increased. This is because the state in which the sweating amount is not changed (i.e., the sweat is not evaporated) after the sweating amount has increased means a state in which a body temperature cannot be reduced by heat of evaporation of the sweat, that is to say, a state in which the transition to the heat stroke state is highly anticipated. Further, the alarm condition may also be met when the determination result of the sweating amount indicates a state in which the sweating amount decreases after indicating the state in which the sweating amount has increased. It is noted that the alarm condition may not be met in the state in which the sweating amount is increasing or in the state in which the sweating amount is decreasing. This is because such states are normal.

In step S707, the output part 48 outputs the alarm. In this way, by outputting the alarm in a state in which the transition to the heat stroke state is highly anticipated, the safety of the test subject 5 can be enhanced. A way of outputting the alarm is arbitrary. For example, a content of the alarm may be varied between the case in which the determination result indicates a non-changed state of the sweating amount after indicating the sweating amount increasing state and the case in which the determination result indicates the sweating amount decreasing state after indicating the sweating amount increasing state. This is because the hazard level is lower in the case in which the sweating amount decreases (i.e., the sweat evaporates) after having sweated (i.e., the sweating amount has increased) than in the case in which the sweating amount does not change. The content of the alarm may be varied such that the attention attraction capability (the volume level of an alarm buzzer, for example) becomes greater as the hazard level becomes greater, for example. In this way, the safety of the test subject 5 can be appropriately enhanced according to the probability (i.e., a hazard level) of the heat stroke by varying the content of the alarm according to the presence or absence of the decrease in the sweating amount after the increase in the sweating amount.

According to the sweat and heart rate determination process illustrated in Fig. 8, the probability of the heat stroke of the test subject 5 can be determined with high accuracy based on the history (a change in a time series) of the calculation result of the sweating amount. With this arrangement, the alarm can be outputted at an adequate timing, and thus the safety of the test subject 5 can be enhanced.

It is noted that, in the process illustrated in Fig. 8, the order in which step S705 and step S707 are performed is arbitrary, and may be reversed. Further, the processes in step S705 and step S707 may be performed simultaneously.

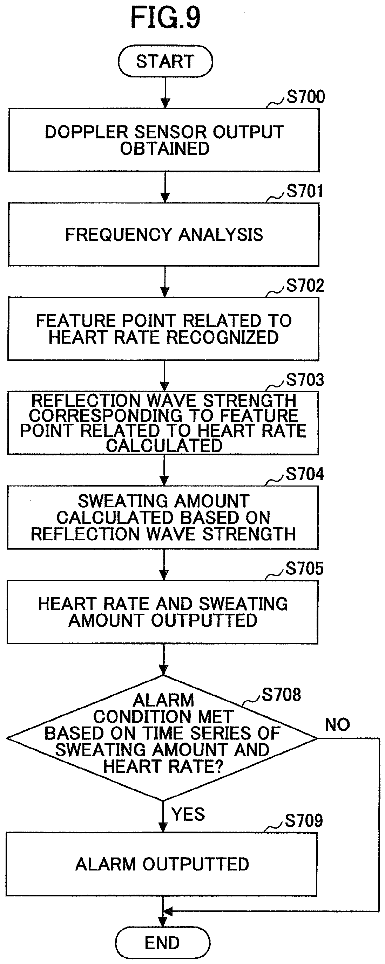

Fig. 9 is a flowchart illustrating another example of a sweat and heart rate determination process executed by the CPU 11 of the sweat and heart rate determination system 10-1. A process routine illustrated in Fig. 8 may be executed repeatedly at a predetermined cycle.

The process illustrated in Fig. 9 differs from the process illustrated in Fig. 7 in that processes after step 708 are added. In the following, the difference is described.

In step S708, the output part 48 determines, based on the calculation result of the heart rate obtained in step S702 and the history (a change in a time series) of the calculation results of the sweating amount obtained in step S704, whether an alarm condition is met. For example, the output part 48 determines "normal" based on a state in which the sweating amount is increasing and the heart rate is within a normal range. The normal range may be fixed or set on a test subject 5 basis. Further, the output part 48 determines "to be monitored" based on a state in which the sweating amount is increasing and the heart rate is within a hazard range. The hazard range is higher than the maximum of the normal range. The hazard range is higher than a predetermined reference value, for example. The predetermined reference value may be fixed or set on a test subject 5 basis. Further, the output part 48 determines "normal" based on a state in which the sweating amount is decreasing and the heart rate is within the normal range. Further, the output part 48 determines "to be monitored" based on a state in which the sweating amount is decreasing and the heart rate is within the hazard range. Further, the output part 48 determines "to be monitored" based on a state in which the sweating amount higher than a reference value continues for a predetermined time and the heart rate is within the normal range. The predetermined reference value related to the sweating amount may be fixed or set on a test subject 5 basis. Further, the output part 48 determines "heat stroke attention" based on a state in which the sweating amount higher than the reference value continues for the predetermined time and the heart rate is within the hazard range. In this case, the output part 48 determines that the alarm condition is met upon the determination of "heat stroke attention". Further, additionally, the output part 48 may determine that the alarm condition is met upon the determination of "to be monitored".

In step S709, the output part 48 outputs the alarm. In this way, by outputting the alarm based on the state (i.e., the state of the determination "heat stroke attention") in which the transition to the heat stroke state is highly anticipated, the safety of the test subject 5 can be enhanced. A way of outputting the alarm is arbitrary. For example, the content of the alarm to be outputted may differ between the case of the determination "heat stroke attention" and the case of the determination "to be monitored". The content of the alarm may be varied such that the attention attraction capability (the volume level of an alarm buzzer, for example) becomes greater as the hazard level becomes greater, for example. In this way, by varying the content of the alarm according to the hazard level, the safety of the test subject 5 can be appropriately enhanced.

According to the sweat and heart rate determination process illustrated in Fig. 9, the probability of the heat stroke of the test subject 5 can be determined with high accuracy based on a combination of the history (a change in a time series) of the calculation result of the sweating amount and the heart rate. With this arrangement, the alarm can be outputted at an adequate timing, and thus the safety of the test subject 5 can be enhanced.

It is noted that, in the process illustrated in Fig. 9, the order in which step S705 and step S709 are performed is arbitrary, and may be reversed. Further, the processes in step S705 and step S709 may be performed simultaneously.

It is noted that, in the processes illustrated in Fig. 8 and Fig. 9, it is determined whether the alarm condition related to the heat stroke is met; however, in addition to or instead of the condition, other conditions may be determined. In other words, the alarm condition is arbitrary, and may be set according to the monitoring purpose (the heat stroke prevention, a heart abnormality monitoring, a drip accident monitoring, a diaper exchange timing monitoring, etc., for example), if appropriate. With this arrangement, when an abnormal sweating amount of the test subject 5 (a large amount of cold sweat at the time of the heart abnormality) is detected, for example, such a fact can be reported to the supervisor of the test subject 5. Further, if the test subject 5 is a patient subject to the drip, the drip accident, in which the drip is removed accidentally, can be detected based on the sudden increase in the moisture state of the wear item 6 due to the drip accident. Also, in this case, the fact can be reported to the supervisor of the test subject 5, for example. It is noted that, in the case of the drip accident monitoring or the diaper exchange timing monitoring, the sweat determination part 44 need not calculate the sweating amount. For example, in the case of the diaper exchange timing monitoring, the sweat determination part 44 may determine the moisture state of the diaper (an example of the wear item 6) to determine, based on the determine moisture state, whether the alarm condition is met (or determine the care timing). In this way, the sweat and heart rate determination system 10-1 can function of watching the state of a care subject (the test subject 5) in a facility such as a nursing care facility, a hospital, etc.

Fig. 10 is a diagram illustrating an example of a way of holding a radio wave transmission/reception part 25.

In the example illustrated in Fig. 10, the radio wave transmission/reception part 25 is held in a holder 8 of a hardness 7. The hardness 7 is on the outer side of the wear item 6. The holder 8 is provided on an outer surface (opposite to the side opposed to the wear item 6) of the hardness 7. The radio wave transmission/reception part 25 thus provided in such a positional relationship transmits the radio waves to the human body of the test subject 5, who wears the wear item 6 and the hardness 7, via the wear item 6 and the hardness 7. The holder 8 may be in any forms. The holder 8 may be in form of a pocket, a belt, Magic Tape (registered trademark), Velcro (registered trademark), etc.

Fig. 11 is a diagram illustrating an example of a hardness 7.

In the example illustrated in Fig. 11, the hardness 7 includes the holder 8 on the inner side (i.e., the side opposed to the human body). The hardness 7 may include pockets for accommodating a water bottle, other sensor devices, a wireless communication device, etc., in addition to the radio wave transmission/reception part 25. The hardness 7 may be in form of a belt or the like, or in form of a vest.

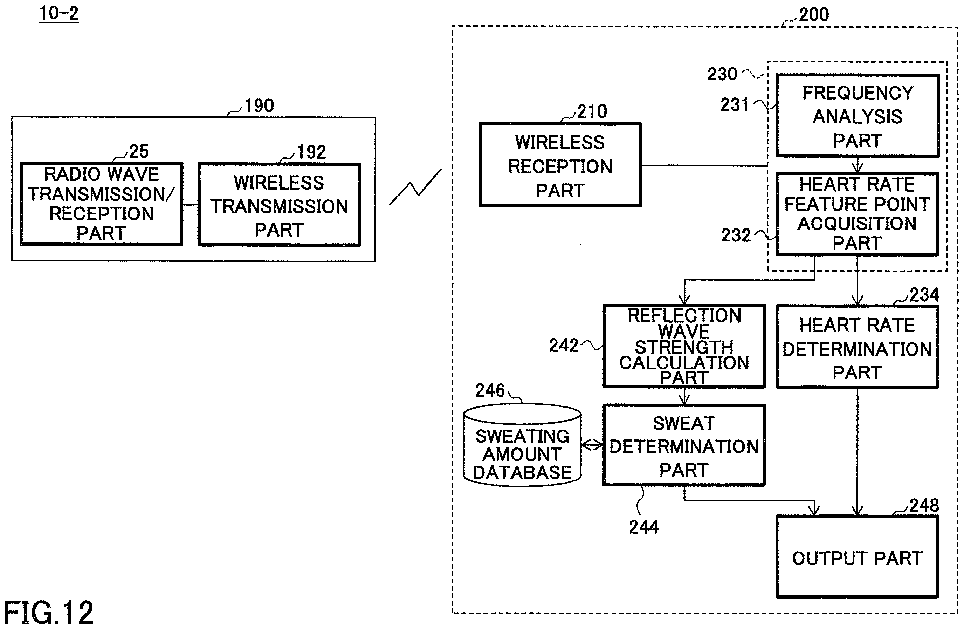

Fig. 12 is a configuration diagram illustrating a sweat and heart rate determination system 10-2 according to another example.

In the example illustrated in Fig. 12, the sweat and heart rate determination system 10-2 includes a sensor unit 190, and a sweat and heart rate determination apparatus 200.

The sensor unit 190 is accommodated in a holder 8 of a hardness 7 (see Fig. 10, and Fig. 11), for example. The sensor unit 190 includes a radio wave transmission/reception part 25 and a wireless transmission part 192. The sensor unit 190 may include hardware resources, such as a CPU, a RAM, a ROM, etc. For example, the sensor unit 190 may have such a hardware configuration in which the display device 22, the display control part 15, the recording media interface 14, the SD card 21, the input/output control part 16, and the input/output device 24 of the sweat and heart rate determination system 10-1 illustrated in Fig. 2 are omitted. In this case, the wireless transmission part 192 can be implemented by the wireless transmission/reception part 26 or a part thereof (a transmission part) illustrated in Fig. 2.

The wireless transmission part 192 transmits the Doppler sensor output obtained by the radio wave transmission/reception part 25 to the sweat and heart rate determination apparatus 200.

The sweat and heart rate determination apparatus 200 is disposed remotely with respect to the sensor unit 190. The sweat and heart rate determination apparatus 200 may be provided in a monitoring center that monitors trainers who are subject to the training and wear the harnesses 7, for example. The sweat and heart rate determination apparatus 200 may be in the form of a server. The sweat and heart rate determination apparatus 200 may have the same hardware configuration as the sweat and heart rate determination system 10-1 illustrated in Fig. 2. However, the recording media interface 14, the SD card 21, the input/output control part 16, the input/output device 24, etc., may be omitted, if appropriate. In the following, it is assumed that the sweat and heart rate determination apparatus 200 may have the same hardware configuration as the sweat and heart rate determination system 10-1 illustrated in Fig. 2.

The sweat and heart rate determination apparatus 200 includes a wireless reception part 210, an analysis part 230, a heart rate determination part 234, a reflection wave strength calculation part 242, a sweating amount determination part 244, a sweating amount database 246, and an output part 248. The analysis part 230 includes a frequency analysis part 231 and a heart rate feature point acquisition part 232. The wireless reception part 210 can be implemented by the wireless transmission/reception part 26 or a part thereof (a reception part) illustrated in Fig. 2. Further, the functions of the analysis part 230, the heart rate determination part 234, the reflection wave strength calculation part 242, the sweating amount determination part 244, and the output part 248 can be implemented by the CPU 11 illustrated in Fig. 2. The sweating amount database 246 can be implemented by the ROM 13 illustrated in Fig. 2. Other auxiliary storage devices such as a HDD, etc., may be used instead of the ROM 13.

The wireless reception part 210 receives the Doppler sensor output from the wireless transmission part 192.

The functions of the analysis part 230, the heart rate determination part 234, the reflection wave strength calculation part 242, the sweating amount determination part 244, and the sweating amount database 246 may be the same as those of the corresponding elements of the sweat and heart rate determination system 10-1 described above, respectively. In other words, the functions of the analysis part 230, the heart rate determination part 234, the sweating amount determination part 244, etc., may be the same as those of the analysis part 30, the heart rate determination part 34, the sweat determination part 44, etc., of the sweat and heart rate determination system 10-1 described above, respectively.

The function of the output part 248 may be the same as the output part 48 of the sweat and heart rate determination system 10-1 described above. However, the output part 248 may output the determination results related to a plurality of the test subjects 5, on a test subject 5 basis. The destination to be outputted by the output part 248 may be a monitor, etc., in the monitoring center, for example. The monitor can be implemented by the display device 22 illustrated in Fig. 2.

According to the sweat and heart rate determination system 10-2 illustrated in Fig. 12, the same effects as the sweat and heart rate determination system 10-1 described above can be obtained.

It is noted that, in the example illustrated in Fig. 12, the wireless transmission part 192 transmits the Doppler sensor output to the sweat and heart rate determination apparatus 200; however, this is not indispensable. For example, the wireless transmission part 192 may transmit the determination result of the heart rate of the test subject 5, and the determination result of the sweating amount of the test subject 5 to the sweat and heart rate determination apparatus 200. In this case, in the sweat and heart rate determination apparatus 200, the analysis part 230, the heart rate determination part 234, the reflection wave strength calculation part 242, the sweating amount determination part 244, and the sweating amount database 246 may be omitted. Further, in this case, the sensor unit 190 may include the analysis part 30, the heart rate determination part 34, the reflection wave strength calculation part 42, the sweat determination part 44, and the sweating amount database 46. Alternatively, from the same viewpoint, the wireless transmission part 192 may transmit intermediate data (frequency analysis results, for example) to obtain the determination result of the heart rate and the determination of the sweating amount of the test subject 5 to the sweat and heart rate determination apparatus 200.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a showing of the superiority and inferiority of the invention. Although the embodiment(s) of the present inventions have been described in detail, it should be understood that the various changes, substitutions, and alterations could be made hereto without departing from the spirit and scope of the invention. Further, all or part of the components of the embodiments described above can be combined.

For example, according to the embodiments described above, the sweat determination part 44 determines the sweating amount from the reflection wave strength based on the information representing the correlation between the reflection wave strength and the absolute humidity. However, as an equivalent variant, the sweat determination part 44 may determine the sweating amount of the human body from the attenuated amount of the reflection wave strength, using information representing the correlation between the attenuated amount of the reflection wave strength and the absolute humidity. In this case, the attenuated amount of the reflection wave strength may be calculated with respect to a reference value of the reflection wave strength. The reference value of the reflection wave strength may be the reflection wave strength obtained in the initial state (in a state in which the sweating amount of the human body is substantially 0, for example). Further, information representing the correlation between the reflection wave strength and a relative humidity or an impedance, instead of the absolute humidity, may be used. For example, the sweat determination part 44 may determine the sweating amount based on information representing the correlation between the reflection wave strength and the relative humidity, and temperature information from a temperature sensor (not illustrated).

Further, in the embodiments described above, the reflection wave strength calculation part 42 calculates, as the reflection wave strength, the peak value related to the frequency of the feature point of the heart rate; however, the reflection wave strength used in the determination process of the sweat determination part 44 may be calculated in other ways. For example, the reflection wave strength calculation part 42 may calculate, as the reflection wave strength, the peak value P0 (see Fig. 5) related to the frequency of the feature point of the respiration, instead of the peak value related to the frequency of the feature point of the heart rate. In this case, the sweat determination part 44 determines the sweating amount of the human body using the peak value P0 related to the frequency of the feature point of the respiration as the reflection wave strength.

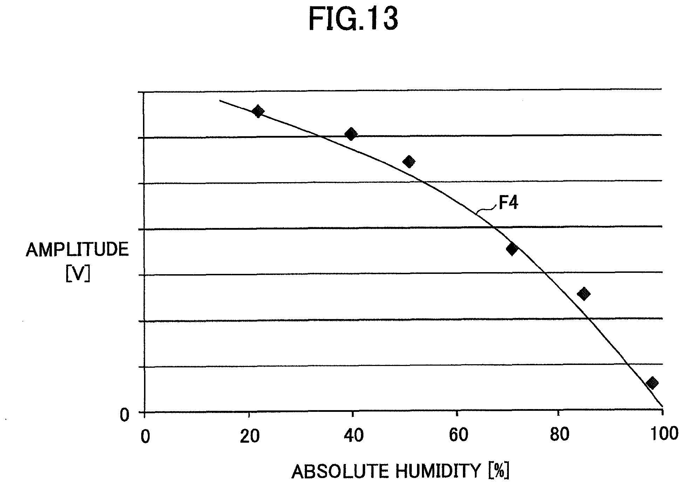

Alternatively, the sweat determination part 44 may determine the sweating amount of the human body based on the moisture sensor output. In this case, the reflection wave strength calculation part 42 calculates, as the reflection wave strength, the amplitude of signal of the reflection waves (i.e., the moisture sensor output) input from the operational amplifier 258 of the radio wave reception part 2. In this case, the reflection wave strength calculation part 42 may calculate, as the reflection wave strength, the amplitude of a waveform of the reflection waves from one peak to another peak. The reflection wave strength calculation part 42 may calculate, as the reflection wave strength, an average of the amplitudes over a predetermined time period. Fig. 13 illustrates the amplitude of the moisture sensor output in the vertical axis. In Fig. 13, the horizontal axis represents the absolute humidity. It is noted that, in Fig. 13, for the sake of explanation, the amplitude is expressed by the voltage value [V] of the signal of the reflection waves. As illustrated in Fig. 13, the amplitude of the reflection waves becomes smaller as the absolute humidity become higher. In other words, the attenuation amount becomes greater as the absolute humidity becomes higher. The amplitude of the reflection waves and the absolute humidity can be associated with each other by a certain relationship F4. The relationship F4 can be derived in advance with a curve fitting using a plurality of items of actual data, as illustrated in Fig. 13. The relationship F4 may be stored in the sweating amount database 46.

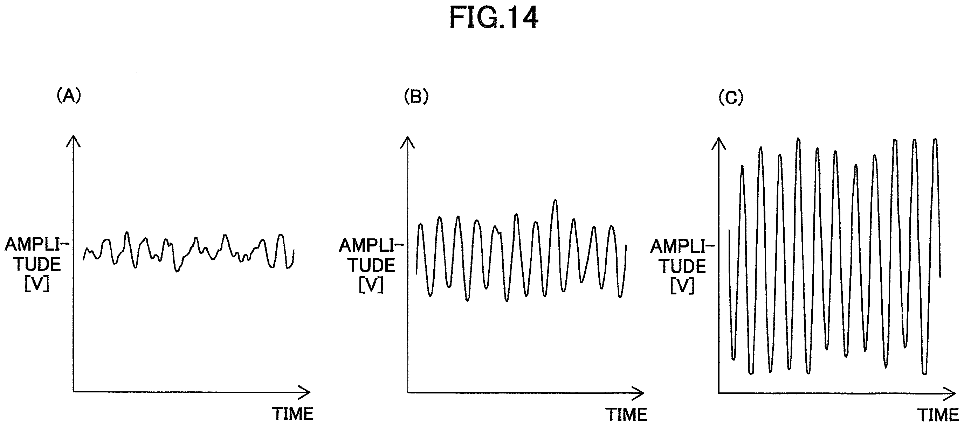

Alternatively, the sweat determination part 44 may determine the sweating amount of the human body based on the Doppler sensor output that is before being subjected to the FFT. In this case, the reflection wave strength calculation part 42 calculates, as the reflection wave strength, the amplitude of signal of the reflection waves (i.e., the Doppler sensor output) input from the operational amplifier 256 of the radio wave reception part 2. In this case, the reflection wave strength calculation part 42 may calculate, as the reflection wave strength, the amplitude of a waveform of the reflection waves from one peak to another peak. The reflection wave strength calculation part 42 may calculate, as the reflection wave strength, an average of the amplitudes over a predetermined time period. Fig. 14 is a diagram illustrating a relationship between absolute humidity and an amplitude of a waveform of a Doppler sensor output. In Fig. 14, (A) illustrates the case of the absolute humidity being 85%, (B) illustrates the case of the absolute humidity being 50%, and (C) illustrates the case of the absolute humidity being 20%. In Fig. 14, a horizontal axis represents a time, and a vertical axis represents an amplitude. In Fig. 14, for the sake of explanation, the amplitude is expressed by the voltage value [V] of the signal of the reflection waves. As illustrated in Fig. 14, similarly, the amplitude of the reflection waves becomes smaller as the absolute humidity become higher. Thus, similarly, the amplitude of the reflection waves and the absolute humidity can be associated with each other with a certain relationship F5 (not illustrated). The relationship F5 can be derived in advance with a curve fitting using a plurality of items of actual data. The relationship F5 may be stored in the sweating amount database 46.

Further, according to the embodiments described above, the output part 48 (and also the output part 248) outputs the information related to the determination result of the sweating amount of the test subject 5; however, the output part 48 may output, as the information related to the determination result of the sweating amount of the test subject 5, information related to a moisture state of the human body or a moisture state of the wear item 6. This is because, as described above, there is a correlation between the moisture state of the human body or the moisture state of the wear item 6 and the sweating amount.

### [Description of Reference Symbols]

1 radio wave transmission part
2 radio wave reception part
6 wear item
10-1, 10-2, sweat and heart rate determination system 10
200 sweat and heart rate determination apparatus
30, 230 analysis part
34, 234 heart rate determination part
44, 244 sweat determination part
48, 248 output part

## Claims

1. A sweat and heart rate determination system, comprising:
a transmitter configured to transmit a radio wave to a human body;
a receiver configured to receive the reflection wave of the radio wave transmitted by the transmitter;
a heart rate determination part configured to determine a heart rate based on a result of a frequency analysis for the reflection wave received by the receiver,
a sweat determination part configured to determine a sweating state of the human body based on a strength of the reflection wave received by the receiver; and
an output part configured to output information related to the heart rate determined by the heart rate determination part, and information related to the sweating state determined by the sweat determination part.

2. The sweat and heart rate determination system of claim 1, further comprising:
an analysis part configured to calculate occurrence timings of a plurality of feature points related to the heart rate based on the result of the frequency analysis for the reflection wave received by the receiver, wherein
the heart rate determination part determines the heart rate based on the occurrence timings of the feature points calculated by the analysis part, and
the sweat determination part determines the sweating state based on the strength of the reflection wave corresponding to the feature points calculated by the analysis part.

3. The sweat and heart rate determination system of claim 1, wherein the transmitter transmits the radio wave to the human body via a wear item that is on the human body, and
the receiver receives the reflection wave that includes a component of reflection of the radio wave at the human body and a component of reflection of the radio wave at the wear item.

4. The sweat and heart rate determination system of claim 3, wherein the wear item is on an upper-body side of the human body.

5. The sweat and heart rate determination system of claim 3, wherein the output part is further configured to output an alarm when a determination result by the sweat determination part indicates a state in which a sweating amount does not change after the sweating amount has increased.

6. The sweat and heart rate determination system of claim 3, wherein the output part varies a content of the alarm to be outputted between a case in which the determination result by the sweat determination part indicates the state in which the sweating amount does not change after indicating the increased sweating amount, and a case in which the determination result by the sweat determination part indicates the state in which the sweating amount decreases after indicating the increased sweating amount.

7. The sweat and heart rate determination system of claim 1, wherein the output part is further configured to change a way of outputting based on a combination of a determination result by the heart rate determination part and a determination result by the sweat determination part.

8. The sweat and heart rate determination system of claim 1, wherein the output part is further configured to determine a state of the human body based on a combination of a determination result by the heart rate determination part and a change in the determination result in a time series by the sweat determination part.

9. The sweat and heart rate determination system of claim 8, wherein determining the state of the human body includes a determination related to a probability of a heat stroke.

10. The sweat and heart rate determination system of claim 9, wherein the output part outputs the alarm when a state in which the sweating amount is higher than a reference value continues for a predetermined time and a state in which the heart rate is higher than a reference value is indicated.

11. The sweat and heart rate determination system of claim 10, wherein the output part varies a content of the alarm to be outputted between a case in which the state in which the sweating amount is higher than the reference value continues for the predetermined time and the state in which the heart rate is higher than the reference value is indicated, and a case in which the state in which the sweating amount is higher than the reference value continues for the predetermined time and the state in which the heart rate is lower than the reference value is indicated.

12. The sweat and heart rate determination system of claim 3, wherein the transmitter and the receiver are held by a hardness that is on an outer side of the wear item.

13. The sweat and heart rate determination system of any one of claims 1 through 12, wherein the radio wave has a frequency in UHF band or in SHF band.

14. The sweat and heart rate determination system of claim 2, wherein the sweat determination part calculates the strength of the reflection wave corresponding to the feature points calculated by the analysis part, and determines the sweating state based on a calculation result of the strength of the reflection wave and information associating the strength of the reflection wave and the sweating state.

15. The sweat and heart rate determination system of claim 1 configured to be carried when the sweat and heart rate determination system is used.

16. A sweat and heart rate determination apparatus, comprising:
a wireless receiver configured to receive a signal representing a reflection wave of a radio wave transmitted to a human body;
a heart rate determination part configured to determine a heart rate based on a result of a frequency analysis for the reflection wave received by the wireless receiver,
a sweat determination part configured to determine a sweating state of the human body based on a strength of the reflection wave received by the wireless receiver; and
an output part configured to output information related to the heart rate determined by the heart rate determination part, and information related to the sweating state determined by the sweat determination part.

17. A method of determining a sweat and heart rate to be executed by a computer, the method comprising:
receiving a signal representing a reflection wave of a radio wave transmitted to a human body;
determining a heart rate based on a result of a frequency analysis for the received reflection wave;
determining a sweating state of the human body based on a strength of the received reflection wave; and
outputting information related to the determined heart rate and information related to the determined sweating state.
